# EUROPEAN PATENT APPLICATION

(11) **EP 1 205 193 A1**
(43) Date of publication of application: **15.05.2002**
(21) Application number: 00870262.3
(22) Date of filing: 07.11.2000
(51) Int. Cl.: A61L 9/03, A01M 1/20

(54) **Easily activatable self-heating device for dispensing volatile materials**

(71) Applicant: THE PROCTER & GAMBLE COMPANY, Cincinnati, Ohio 45202 (US)
(72) Inventor: Blenkiron, Peter, Egham, Surrey TW20 8XB (GB); Curtis, Terence Graham, High Wycombe, Bucks HP11 1JN (GB); Burrowes, Lee, Woking, Surrey GU21 4PR (GB); Metzger-Groom, Sabine Ursula, Morpeth, Northumberland NE61 2DJ (GB)
(74) Representative: Canonici, Jean-Jacques

(57) **Abstract**

Disclosed are devices for dispensing volatile materials such as fragrances into the atmosphere surrounding each such device. Release of volatile materials from a volatilization zone in such a device is promoted by the generation of heat via the non-combustive oxidation reaction of thermogenic materials held within a heating zone in the device. Both the volatilization zone and the heating zone are provided with hermetic sealing means which, prior to device activation, keep the contents of the volatilization and heating zones separate from each other and from the surrounding atmosphere. Such sealing means can be ruptured to expose the thermogenic materials to air to thereby initiate heat generation and to expose the volatilization zone to the surrounding atmosphere to thereby release the volatile materials therefrom.

## Description

### Technical Field/Field of the Invention

The present invention relates to a self-heating device for dispensing volatile materials such as fragrances into the atmosphere surrounding the device.

### Background of the Invention

Devices for providing and dispensing volatile materials are well-known in the art. Such devices can include room freshners or fragrancing articles, vaporizers for humidification or dispensing and dispersal of therapeutic vapors, incense sticks, fragrancing or insect-repelling candles, and the like. Such devices generally involve utilization of some source of heat which promotes the volatilization of the materials to be dispensed and, of course, some source of the volatile or volatilizable materials themselves.

Some fragrancing devices, such as candles and incense sticks, involve use of an open flame or an active combustion reaction to provide the source of heat which promotes volatilization. Besides the obvious drawbacks of the hazards of using such types of heat sources, arrangements which involve flames or combustion are not especially portable and cannot be used, for example, in automobiles or around flammable materials.

Other types of dispensing devices for volatiles use electrical energy as a heat source. Plug-in room air-fresheners, for example, are commercially marketed under the tradenames *Ambi-Pur* and *Glade.* The amount of electrical energy required to operate devices of this type renders such devices relatively non-portable.

Some articles and devices for emitting or dispensing volatile materials employ non-combustive thermogenic materials as a heat source useful for promoting volatilization of the ingredients to be emitted or dispensed. Villamarin; PCT Patent Application WO 91/07996; Published June 13, 1991, for example, discloses a self-contained fragrance delivery package, comprising a fluent volatilizable fragrance material and an air-activatable chemical heat source. Disclosed configurations include a layered pad with covered perforations which can be removed to expose the heat source to air and activate the pad. Other dispensing devices using a non-combustive chemical heat source are disclosed in Takei; U.S. Patent 4,330,506; Issued May 18, 1982; Koiso et al; U.S. Patent 4,516,564; Issued May 14, 1985 and Arao; Japanese Patent Application 63175771; Published January 26, 1990.

Such devices in general are not necessarily readily consumer activatable and furthermore may have problems with efficient transfer of heat to the volatilizable materials or effective discharge of the volatile materials from the device or article into the surrounding atmosphere. Such heat transfer and/or discharge problems may be caused by undesirable interaction between the chemical heat source materials and the materials to be volatilized. Given this situation, there is a continuing need to provide dispensing devices in forms which are safe, portable, readily consumer activatable and especially effective at dispensing the desired volatile materials in suitable amounts and over an acceptably prolonged period of time.

### Summary of the Invention

The present invention is directed to a dispensing device for emitting volatile materials into the atmosphere surrounding the device. The invention further relates to the use of such a device for emitting volatiles such as fragrances.

In its broadest sense, the devices herein comprise at least one volatilization zone containing volatile and/or volatilizable materials and at least one heating zone containing air-activatable thermogenic materials. The volatilization zone is juxtaposed with and in thermodynamic communication with the heating zone. Both the volatilization zone and the heating zone are provided with hermetic sealing means which prevent contact of the contents of these two zones with each other, and with the outside atmosphere, prior to activation of the device by the rupturing of the sealing means.

In a preferred configuration, the dispensing devices herein comprise an insert, which itself comprises two separate zones, and a holder for this insert. One zone of the insert is a substantially rigid volatilization zone which contains volatile or volatilizable materials. The other zone of the insert is a heating zone which contains air-activatable thermogenic materials. This volatilization zone and the heating zone are juxtaposed and in thermodynamic communication with each other within the insert.

Both the volatilization zone and the heating zone of the insert are sealed from the atmosphere surrounding the insert by rupturable sealing means such as, for example, aluminum foil. Such sealing means can be ruptured to expose both zones to the atmosphere. Such exposure activates the thermogenic materials within the heating zone and releases the volatile materials from the volatilization zone into the atmosphere.

In the preferred device configuration, the holder for the insert comprises both a substantially rigid base and a substantially rigid cover for the base. The base and/or the cover of the holder will also comprise means, such as relatively sharp protrusions, which can be used to rupture the seals of the zones of the insert. These rupturing means can be activated by the user of the dispensing device to rupture the insert seals, and this serves to activate the dispensing device. Upon activation, the heat generated in the heating zone enhances volatilization of the materials in the volatilization zone and promotes their emission into the surrounding atmosphere.

### Brief Description of the Drawings

Figure 1 is a view in perspective of a fragrance dispensing device according to the present invention, prior to the activation of the device.

Figure 2 is a view in perspective of a fragrance dispensing device according to the present invention after the device has been activated.

Figure 3 is a section view of the pre-activated device of Figure 1.

Figure 4 is an exploded view of the insert element of a device according to the present invention.

### Detailed Description of the Invention

The dispensing device of the present invention comprises both volatilization and heating zones. These zones are preferably found within an insert which is carried and held within a two-part holder. All of the elements of the various dispensing device configurations herein are described in detail as follows :

### A) Insert

An insert is the component of the preferred dispensing device which holds both the volatile or volatilizable materials to be emitted from the device and the thermogenic materials which serve to provide the thermal energy to help volatilize such materials. Both the volatile or volatilizable materials which are held within the volatilization zone and the thermogenic materials which are held within a heating zone are sealed from the atmosphere surrounding the insert until the device is activated by rupturing the seals of these zones and exposing the contents of the insert to the outside air.

### i) Volatilization Zone

One essential component of the devices, and preferably the insert, herein is the volatilization zone. It is the volatilization which holds the volatile or volatilizable materials which the dispensing device of this invention is designed to emit. The volatilization zone can be of any shape or configuration but will generally be substantially rigid.

Preferably the volatilization zone will have a volume of from 1 cm³ to 50 cm³, more preferably from 3 cm³ to 15 cm³. It will also preferably be elongated, having a major dimension of from 1 cm to 15 cm, more preferably from 2 cm to 10 cm. Preferably the volatilization zone will be configured within the device, e.g., within the insert, such that the major dimension of the volatilization zone is generally in a vertical position as the insert is held within the dispensing device.

Frequently the volatilization zone, e.g., within the insert, will be generally cylindrical in configuration. Alternatively, the volatilization zone can have a tapered configuration. Preferably the volatilization zone will be openable to the atmosphere at both of its ends. Concurrently filed European Patent Application No. ___________ (P&G Case No.CM-2467F) is directed to devices having a volatilization zone of this configuration

In the cylindrical configuration, the volatilization zone will typically have a circular cross-sectional area which ranges from 1 cm² to 10 cm² more preferably from 2 cm² to 6 cm². In the tapered configuration, the volatilization zone will generally have a smaller circular cross-sectional area at or near the top of the zone than at or near the bottom of the zone. For the tapered configuration, the circular cross-sectional area at or near the top of the volatilization zone will typically range from 0.5 cm² to 5 cm², more preferably from 1 cm² to 3 cm², and the circular cross-sectional area at or near the bottom will typically range from 1 cm² to 10 cm², more preferably from 2 cm² to 6 cm². In the tapered configuration, the volatile or volatilizable materials tend to move through the volatilization zone faster at or near the top by virtue of the effect of the Bernulli principle. This helps to boost the effectiveness of the volatilized material emission into the atmosphere surrounding the device.

The volatilization zone, as with the rest of the elements of the dispensing device herein, can be made of any suitable material which provides the requisite rigidity, strength, heat resistance/heat conductivity and compatibility with the materials to be held therein. Typically thermoplastic or thermosetting materials such as polyethylene, polypropylene, polystyrene, acrylic resins, and the like, may be utilized to form the volatilization zone, e.g., within the insert.

The volatilization zone will contain the volatile and/or volatilizable materials which are to be emitted from the device upon rupturing of the volatilization zone sealing means and exposure of this zone to this surrounding atmosphere. Volatile materials are those materials which may be largely in the vapor phase at ambient temperature (e.g. 20°C). Volatilizable materials are those which may be completely or partially in a liquid or solid phase at ambient temperature but which can be vaporized by the heat generated by the thermogenic materials in the heating zone of the devices herein.

Suitable volatile or volatilizable materials are those which can act as fragrances, insect repellents, insecticides and materials which are or which produce therapeutic vapors. Preferred volatile or volatilizable materials are fragrances which can include any odoriferous materials having a vapor pressure of at least 17.5 mmHg @ 20 °C. Generally, the devices herein will contain from 0.1 gram to 10 grams, more preferably from 0.5 gram to 3 grams, most preferably from 1 gram to 2 grams of the volatile or volatilizable materials sealed until activation within the volatilization zone.

The volatile or volatilizable materials may be placed into the sealed volatilization chamber in neat form, or they may be held in the volatilization zone in combination with a liquid or solid carrier substrate. Any material which will absorb, adsorb, or otherwise hold or carry the volatile or volatilizable material until the volatilization zone is exposed to the atmosphere can be employed as carrier or carrier substrate. Especially suitable carriers can include solid absorbent materials such as felt or cellulosics including paper, cotton, airfelt, and the like. Other carrier include porous polyolefin materials. An especially preferred carrier for materials to be held in the volatilization zone is the porous polypropylene material marketed under the tradename, Accurel™.

### ii) Heating Zone

The volatilization zone herein is juxtaposed and in thermodynamic communication with at least one heating zone containing air-activatable thermogenic materials. The heating zone, like the volatilization zone, can be constructed of any suitable material which will hold thermogenic materials and be compatible with such materials and the heat generated thereby when the devices herein are activated. Preferably the heating zone will have a volume of 10 cm³ to 100 cm³, more preferably from 20 cm³ to 40 cm³.

In preferred embodiments wherein the volatilization zone is generally cylindrical, the heating zone will be of generally annular configuration, surrounding the volatilization zone, with the generally cylindrical volatilization zone running through the core of the heating zone.

The heating zone will contain thermogenic materials which produce thermal energy via a non-combustive oxidation reaction upon contact with oxygen-containing gas such as for example upon exposure to and contact with air. Typically such materials are those used to bring about the exothermic oxidation of iron and are used in particulate form. Generally the heating zone will contain from 10 grams to 50 grams, more preferably from 15 grams to 30 grams, of the thermogenic material combination.

A preferred combination of air-activatable thermogenic materials comprises from 30% to 80% by weight of the combination of iron powder, from 3% to 25% by weight of the combination of carbon particles, from 0.5% to 10% by weight of the combination of a metal salt such as alkali metal or alkaline earth metal salts, and from 1% to 40% by weight of the combination of water. Such thermogenic material combinations may also contain an additional water-holding particles (e.g., vermiculite, porous silicates) present to the extent of from 0.1% to 30% by weight of the combination. Other optional ingredients in the thermogenic material combination include oxidation reaction enhancers (chromium, manganese, copper), hydrogen gas inhibitors (sodium hydroxide, sodium thiosulfate), fillers (cellulosics), anti-caking agents (tricalcium phosphate, sodium silicoaluminates), thickeners (starches) and surfactants. Thermogenic materials of the type useful in the heating zone of the inserts herein are described in greater details in Burkett et al; U.S. Patent 5,918,590; Issued July 6, 1999.

The thermogenic materials used in the heating zone should, upon activation, be capable of producing a temperature within the heating zone of at least 60 °C within a period of from 3 to 7 minutes after contact of air with the thermogenic materials is initiated. Preferably, a temperature of at least 50 °C will be maintained for a period of at least 180 minutes, more preferably at least 240 minutes, after activation of the device.

The dispensing devices herein operate most effectively if the devices, or inserts therefor, comprise means for enhancing exposure of the thermogenic materials therein to activating air upon rupturing of the sealing means of the heating zone. For purposes of this invention, exposure of the thermogenic materials to air is "enhanced" if thermogenic material is contacted with more air than is provided by the simple exposure of the outside surface of the themogenic material when the heating zone seal is ruptured. Thus, for example, air exposure to the thermogenic materials can be enhanced by providing the thermogenic materials in particulate form and packed in a manner such that the mass of thermogenic materials has a porosity which is suitable to permit permeation of air thereinto. This can be accomplished by altering the particle size of the thermogenic materials or by providing structurants or spacers (e.g., starches, glass beads, styrofoam particles, etc.) within the mass of thermogenic material particles. Alternatively, or in addition, air inlet channels such as perforated tubes or screens, can be positioned within and preferably throughout the mass of thermogenic materials.

The thermogenic materials in the heating zone are preferably held and constrained within this zone by air-permeable holding means. Such holding means serves to prevent the generally solid, e.g., granular, thermogenic materials from being removed from the device, either purposefully or unintentionally, once the sealing means of the heating zone have been ruptured and the heating zone is thereby opened to the outside atmosphere. Such air-permeable holding means, can comprise, for example, fabric- or polymer-based mesh materials having mesh openings, e.g., apertures, therein which are large enough to permit suitable flow of air therethrough but small enough to prevent significant amounts of the solid thermogenic material from passing through the mesh.

### iii) Sealing Means

Both the volatilization zone and the heating zone herein are hermetically sealed from the outside atmosphere by sealing means. Such sealing means serve to prevent the escape of the volatile or volatilizable materials from the volatilization zone, and to prevent the activation of the thermogenic materials in the heating zone, until the sealing means are ruptured. The sealing means also keep the contents of the volatilization zone and the heating zone out of contact with each other.

Preferably the sealing means for both volatilization zone and the heating zone-will be located at more than one place on each zone. In a preferred configuration for the inserts, wherein both the volatilization and heating zones are elongated and held in a generally vertical position, the rupturable sealing means will be positioned at both the upper and lower end of each zone. In this way, when the sealing means are ruptured at both ends of these zones, airflow into and through each zone is promoted.

Sealing means can comprise any kinds of materials which are both air and liquid impervious when intact but which can be readily ruptured when the dispensing device is to be activated. Suitable sealing means for both volatilization and heating zones include any type of single ply or laminated film, foil or sheet which has the requisite type of impermeability and rupturability characteristics. Laminated aluminum foil is especially preferred as a sealing means useful in the devices of this invention. The sealing material may, in fact be co-laminated with holding means for the thermogenic material, e.g., with flexible mesh. In this setup, the rupturing means of the holder may be configured to rupture the foil sealing means but not the flexible mesh holding means laminated to the foil.

### B. Holder

The inserts of the preferred device configuration, as described hereinbefore, are held with the volatilization chamber in a generally upright vertical position by a holder component for the insert. In the preferred devices herein, the holder comprises a substantially rigid base and a substantially rigid cover for the base. The shape of the holder cover and holder base will, of course, depend upon the shape and configuration of the insert which is to fit inside the holder. For inserts which are generally cylindrical in configuration, the base and cover comprising the holder will generally be cylindrical or spherical.

The holder element of the preferred devices herein must have means positioned on the cover or base, and preferably on both, for rupturing the sealing means used for the chambers or zones of the insert. Such rupturing means must be consumer activatable, i.e., when the user of the dispensing device wants to activate it, the user must be able to cause the rupturing means to puncture or rupture the sealing means on the insert zones so that exposure to air of the thermogenic materials begins the heat generation process and exposure to air of the volatilizable materials begins the process of dispensing these materials into the surrounding atmosphere. Generally the seal rupturing means associated with the base and/or cover simply comprises rigid and relatively sharp protrusions which can be forced against the sealing means until rupture of the sealing means occurs.

In one preferred configuration, the covering base of the holder are slideably affixed to each other such as the user of the device can activate it by pushing the cover and base closer together. As illustrated in the drawing hereinafter, this action serves to push the puncturing protrusions which are part of both base and cover into and through the seals of the insert which is held inside the slideably closeable base and cover.

The cover and base elements of the holder may also be configured to control or alter the flow of air into and through the dispensing device once the seals have been ruptured. This can be accomplished by altering the size of the air inlet openings and/or the configuration and number of seal puncturing means. The cover and base elements of the holder may also be rotatable or otherwise moveable in relation to each other in order to vary the size of the air inlet openings.

The holder may also preferably be constructed of thermochromic material such that at least a portion of the outside surface of the holder changes color upon heating. In this manner, the device can be constructed of a material which changes color when the device is activated and the holder heats up as a result of thermal energy generation in the heating zone(s). This provides a signal to the user of the device that the device has been activated and is generating heat and emitting volatiles.

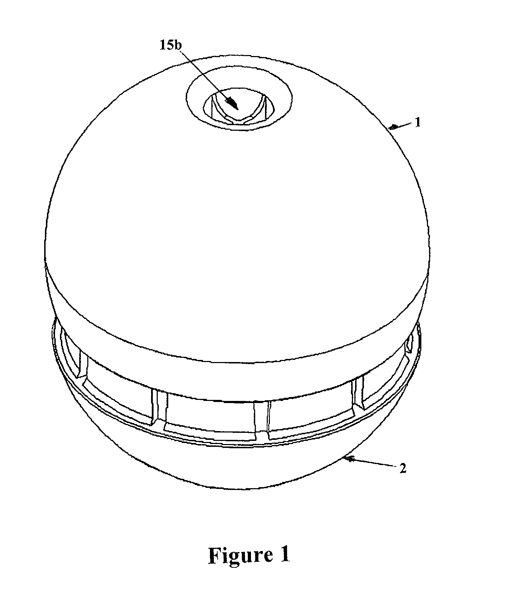

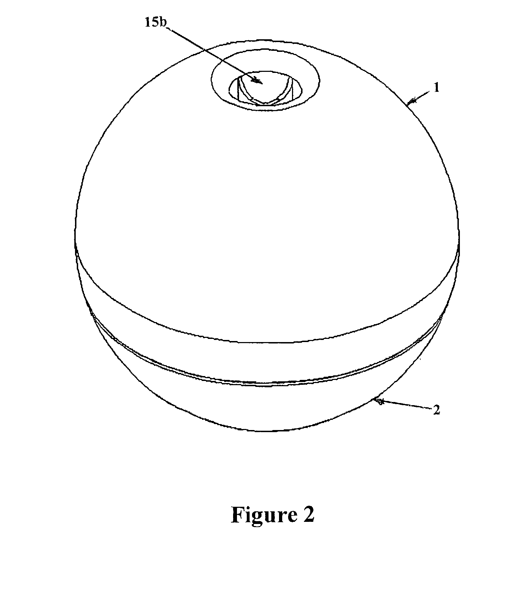

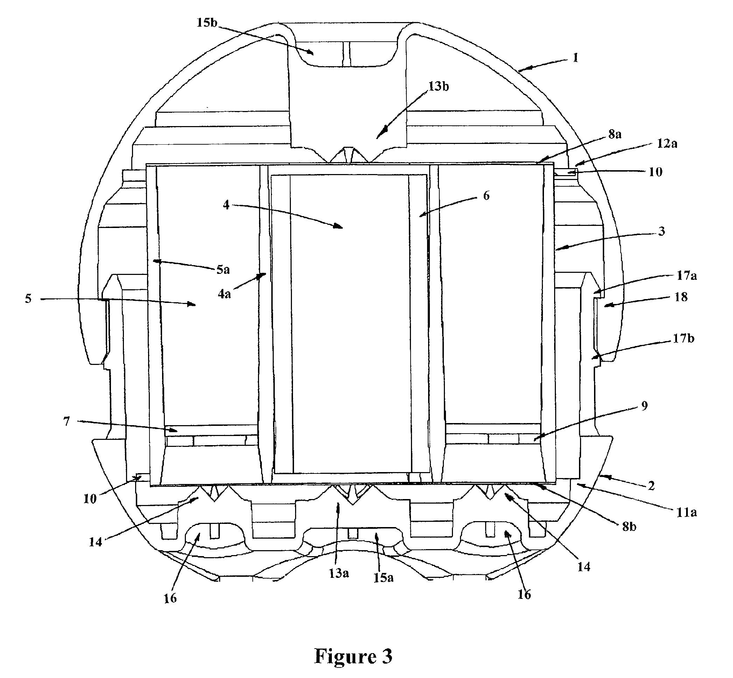

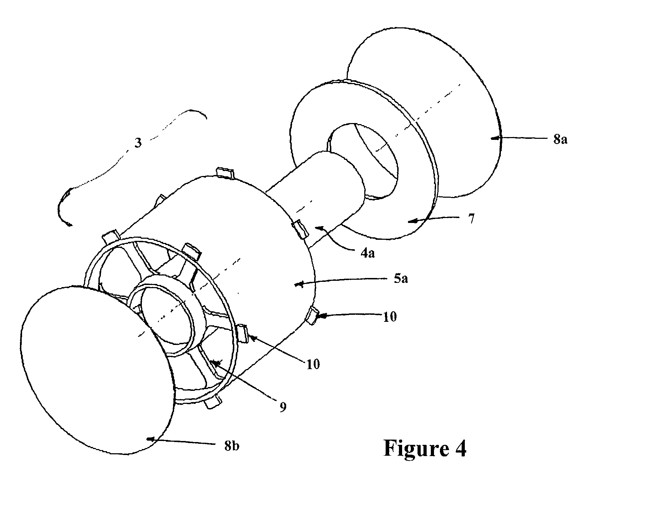

A highly preferred dispensing device according to the present invention is one having a cylindrical insert held within a spherical holder. Such a device depicted in Figs. 1-4 of the drawings. Figure 1 is an external view of the dispensing device in its preactivated condition. Figure 2 is an external view of the dispensing device which has been activated by pushing the holder pieces together. Figure 3 is a cross-sectional view of the device in its preactivated condition. Figure 4 is an exploded view of the insert component of the dispensing device.

In the several figures there is shown the dispensing device holder comprising a cover **1** which snaps onto a base **2.** Inside the cover and base is a generally cylindrical insert **3.** The insert comprises a cylindrical volatilization zone **4** along the axis of the insert as defined by cylinder wall **4a.** The volatilization zone is surrounded by an annular chamber comprising the heating zone **5** as defined by outside cylindrical wall **5a.** Positioned within the volatilization zone is a carrier tube **6** of Accurel onto which is absorbed an effective amount of a perfume material (not shown). Positioned within the annular heating zone is an effective amount of thermogenic materials (not shown) which are held in place within the heating zone by air preamble wad **7.** Prior to device activation, both the volatilization zone and the heating zone, with the volatile/volatilizable materials and thermogenic materials therein respectively, are sealed at both the top via a top seal **8a** and the bottom via bottom seal **8b.**

The insert is provided with a support spoke **9** which supports the permeable wad **7.** The support spoke has breakable support lugs **10** which rest on the base shoulder **11a** and support the cover at shoulder **16** before the device is activated. The base is provided with a central bottom seal penetrating protrusion **13a** which is positioned to rupture the bottom seal and open the volatilization zone. The base is also provided with four perimeter bottom seal penetrating protrusions **14** positioned to also puncture the bottom seal and open the heating zone. The base is also fitted with a bottom center air inlet opening **15a** and other bottom perimeter air inlets **16** to provide exposure of the volatilization zone and heating zone, respectively, to the atmosphere upon rupture of the bottom seals when the user activates the device.

The cover is also provided with a central top seal penetrating protrusion **13b** and may contain other protrusions (not shown) which are positioned to also puncture the top seal and open the volatilization zone further. The cover also includes a top outlet opening **15b** through which the volatile or volatilized materials are emitted to the outside atmosphere upon activation of the dispensing device. The base is also provided with a clip feature formed by ridges **17a** and **17b** into which snaps the cover clip feature **18.** The clip features keep the cover and base apart, and the seals unpunctured, prior to activation of the device.

### Mode of Operation

### Prior to activation:

The insert **3** is suspended inside the holder cover **1** and holder base **2.** The cover and base are kept apart to prevent rupturing of the insert seals **8a** and **8b** by interacting with clip feature **17a, 17b** and **18** and breakable support lugs **10.** A further anti-activation device (not shown in the drawings) could also be used to prevent movement of the cover and base towards each other. Such anti-activation devices could include a clip or shrink sleeve or tamper band.

### To activate:

The cover and base **1** and **2** are pushed together by overcoming clips **17a** and **17b** and **18,** breaking or deforming lugs **10** and piercing seals **8a** and **8b** via the cutter protrusions **13a** and **13b** and **14.** This results in air being allowed to flow through the volatilization zone **4** and the volatile, e.g., fragrance, carrier insert **6** and also into the heating zone **5.** This results an exothermic reaction taking place in the heating zone **5,** which results in heat transfer from the heating zone into the volatilization zone. This, in turn, creates convection currents in the volatilization zone and enhances volatilization of the volatilizable materials which are discharged into the atmosphere through opening **15a.** The clips prevent the cover from being removed from the holder base and ensure that the rupturing protrusions stay in place keeping air channels **15a** and **16** open.

## Claims

1. A dispensing device for emitting volatile materials into the atmosphere surrounding the device, said device being **characterized in that** it comprises:
A) at least one volatilization zone containing volatile and/or volatilizable materials, said volatilization zone being juxtaposed with and in thermodynamic communication with:
B) at least one heating zone containing air-activatable thermogenic materials;
wherein both said volatilization zone and said heating zone are provided with hermetic sealing means which prevent contact of the contents of said zones with each other and with the outside atmosphere prior to activation of said device by the rupturing of said sealing means.

2. A dispensing device for emitting volatile materials into the atmosphere surrounding the device, said device being **characterized in that** it comprises:
A) an insert comprising:
i) a substantially rigid volatilization zone containing volatile and/or volatilizable materials, said volatilization zone being juxtaposed with and in thermodynamic communication with:
ii) a heating zone containing air-activatable thermogenic materials;
both said volatilization zone and said heating zone being sealed from the atmosphere surrounding said insert via sealing means which can be ruptured to expose both zones to the atmosphere and to thereby activate the thermogenic materials within said heating zone and release volatile materials from the volatilization zone into the atmosphere; and
B) a holder for said insert, said holder comprising:
i) a substantially rigid base; and
ii) a substantially rigid cover for said base;
wherein said base, said cover, or both, comprise means for rupturing the sealing means of the volatilization and heating zones of said insert.

3. A device according to Claim 2 wherein said holder for the insert carries the insert in a manner which maintains the integrity of the sealing means for the zones of the insert until rupturing of said sealing means is desired.

4. A device according to Claim 3 wherein the insert is held within the holder by means of breakable lugs which comprise part of the insert, the holder, or both

5. A device according to any of Claims 2 to 4 wherein said rupturing means associated with said base and/or cover of said insert can be activated by the user of the device to rupture said sealing means of the volatilization and/or heating zones of said insert.

6. A device according to any of Claims 2 to 5 wherein said base and/or said cover of said holder can be moved in relation to said insert in a manner which causes the rupturing means associated with said base and/or cover to rupture the seals of said volatilization and/or heating zones of said insert.

7. A dispensing device for emitting volatile materials into the atmosphere surrounding the device, said device being **characterized in that** it comprises:
A) an insert comprising:
i) a substantially rigid volatilization zone containing volatile and/or volatilizable materials, said volatilization zone being juxtaposed with and in thermodynamic communication with:
ii) a heating zone containing air-activatable thermogenic materials;
both said volatilization zone and said heating zone being sealed from the atmosphere surrounding said insert via sealing means which can be ruptured to expose both zones to the atmosphere and to thereby activate the thermogenic materials within said heating zone and release volatile materials from the volatilization zone into the atmosphere; and
B) a holder for said insert, said holder comprising:
i) a substantially rigid base; and
ii) a substantially rigid cover for said base;
wherein said base and/or said cover of the holder are configured to control the flow of air through said volatilization and/or heating zones of the insert once the sealing means for said zones are ruptured.

8. A device according to any of Claims 2 to 7 wherein said rupturing means comprise projections associated with said base and/or said cover of the holder, which projections are capable of puncturing said sealing means of the volatilization and/or heating zones of the insert upon the application of force onto said base and/or cover.

9. A device according to any of Claims 2 to 8 wherein said holder is constructed of thermochromic material such that at least a portion of the outside surface of said holder changes color when said device is activated and thermal energy is produced therein.

10. An insert for a dispensing device for volatile materials, said insert being **characterized in that** it comprises:
A) a substantially rigid volatilization zone containing volatile and/or volatilizable materials, said volatilization zone being juxtaposed with and in thermodynamic communication with:
B) a heating zone containing air-activatable thermogenic materials;
both said volatilization zone and said heating zone being sealed from the atmosphere surrounding said insert via sealing means which can be ruptured to expose both zones to the atmosphere and to thereby activate the thermogenic materials within said heating zone and release volatile materials from the volatilization zone into the atmosphere.

11. A device or insert according to any of Claims 1 to 10 wherein said sealing means comprise an aluminum foil-based laminate.

12. A device or insert according to any of Claims 1 to 11 wherein the volatile or volatilizable materials within said volatilization zone are selected from the group consisting of fragrances, insect repellents, insecticides and materials which are or which produce therapeutic vapors.

13. A device or insert according to any of Claims 1 to 12 wherein the thermogenic materials within said heating zone are capable of generating thermal energy via an iron oxidation reaction.

14. A device or insert according to Claim 13 wherein said thermogenic materials comprise a combination of iron, carbon, water and inorganic salts.

15. A device or insert according to any of Claims 1 to 14 wherein said thermogenic materials are held or constrained within said heating zone by air-permeable holding means.

16. A device or insert according to any of Claims 1 to 15 wherein said thermogenic materials are capable of producing a temperature within said heating zone of at least 60 ° C within a period of 3 to 7 minutes after contact of air with said thermogenic materials is initiated.

17. A device or insert according to any of Claims 1 to 16 wherein said volatilization zone has a generally vertical major dimension which ranges in length from 1 cm to 15 cm.

18. A device or insert according to Claim 17 wherein said volatilization zone is generally cylindrical.

19. A device or insert according to Claim 17 wherein said volatilization zone is generally of tapered configuration such that its horizontal cross-sectional area at or near the top of its vertical dimension is smaller than its horizontal cross-sectional area at or near the bottom of its vertical dimension.

20. A device or insert according to any of Claims 1 to 19 wherein said volatilization zone has a horizontal cross-sectional area which is generally circular.

21. A device or insert according to Claim 20 wherein said heating zone is of generally annular configuration and completely surrounds said volatilization zone.

22. A device or insert according to any of Claims 1 to 21 which further comprises means for enhancing exposure of said thermogenic materials to air upon activation of said device by the rupturing of said sealing means.

23. A device or insert according to Claim 23 wherein said air exposure enhancing means are selected from the group consisting of:
A) utilizing thermogenic materials which are in particulate form and which are packed into a mass having a porosity such that air will permeate into the mass of thermogenic particulate materials:
B) providing at least one air inlet channel within a mass of said thermogenic materials; and
C) combinations of said air exposure enhancing means.

24. Use of a dispensing device or insert therefor according to any of Claims 1 to 21 by initiating contact of said thermogenic materials within said device or insert with air.
